# EUROPEAN PATENT SPECIFICATION

(11) **EP 3 348 307 B1**
(45) Date of publication and mention of the grant of the patent: **02.03.2022**
(21) Application number: 18151315.1
(22) Date of filing: 12.01.2018
(51) Int. Cl.: A61Q 19/00, A61K 31/205, A61K 31/56, A61P 17/00, A61P 17/06

(54) **COMPOSITION COMPRISING CARNITINE, SODIUM CHOLATE, SODIUM ACETATE AND OPTIONALLY SILVER FOR USE IN THE TREATMENT OF PSORIASIS**
ZUSAMMENSETZUNG MIT CARNITIN, NATRIUMCHOLAT, NATRIUMACETAT UND GEGEBENENFALLS SILBER ZUR VERWENDUNG IN DER BEHANDLUNG VON PSORIASIS
COMPOSITION COMPRENANT DE LA CARNITINE, DU CHOLATE DE SODIUM, DE L'ACÉTATE DE SODIUM ET ÉVENTUELLEMENT DE L'ARGENT POUR UTILISATION DANS LE TRAITEMENT DU PSORIASIS

(30) Priority: 16.01.2017 IT 201700004019
(43) Date of publication of application: 18.07.2018
(73) Proprietor: GK Pharma Consultants SA, 6830 Chiasso (CH)
(72) Inventor: MAGNI, Enrico, 6830 CHIASSO (CH)
(74) Representative: Bianchetti & Minoja SRL

(56) References cited:
- EP-A1- 2 946 765
- WO-A1-2015/071837
- WO-A2-03/005980
- DE-A1- 19 522 694
- DE-A1-102006 021 198

## Description

### Field of invention

The present invention relates to a pharmaceutical composition comprising carnitine, sodium cholate, sodium acetate and optionally silver for use in the treatment of psoriasis.

### Technical background

In the first 24 hours after injuries to the skin and mucosa, such as infections, bruises, haematomas, irritations, grazes, wounds and burns, an inflammatory reaction takes place. In skin lesions full of blood clots containing platelet aggregates, erythrocytes and leucocytes trapped in a fibrin lattice, blood vessel vasoconstriction factors are initially released to prevent further bleeding.

Subsequently, the increased blood supply due to vasodilation which carries leucocytes gives rise to subcutaneous oedema, high hydrostatic pressure, swelling, warmth and painful distension of the layer of epidermal cells or breakage of the skin.

If the skin is unbroken, blisters may form, as in the case of herpes zoster and burns, and inflammatory exudates can appear on the surface of the lesion.

Skin breakage can lead to major losses of fluid, and energy substrates (carbohydrates, lipids, proteins and biological effectors such as cytokines, trace elements, etc.) significantly alter the chemical composition of the internal environment at local level.

To ensure pain reduction and prevent a cell repair factor deficiency without a qualitative and quantitative variation in wound-healing, reducing the exudate must be viewed as a therapeutic act of primary importance. In this context, the treatment consists of antiseptic cleansing of the surface by means of a light compression bandage. The purpose of said compression is to stabilise and rapidly correct the inadequate nutrient composition in the skin lesion.

Psoriasis is an inflammatory disease of the skin, which is generally chronic. Although its etiology is not yet fully understood, it involves autoimmune, genetic, infectious and environmental factors (skin traumas or pharmacological treatments). Psoriasis commonly appears in the form of areas of thickened scaly skin, mainly located on the hands, feet, elbows, knees and scalp.

Therapy generally involves local treatment with steroids such as betamethasone, calcipotriol, keratolytic agents (salicylic acid, urea) or emollients (vaseline) to reduce the dryness of the skin, or tar. However, despite their efficacy, corticosteroids notoriously present a number of adverse effects. Tar must also be used with caution in view of its irritant action.

French patent 2,850,276 describes a pharmaceutical composition containing betaine and steroidal surfactants for the treatment of dermatitis, mucosal lesions and slow-healing sores and ulcers. However, said composition is ineffective in the treatment of disorders such as psoriasis, vitiligo and rosacea.

### Description of the invention

It has now been found that the combination of carnitine, sodium cholate, sodium acetate and optionally silver has a favourable action in the treatment of psoriasis. The present invention therefore relates to a composition comprising
a) carnitine,
b) sodium cholate,
c) sodium acetate,
   and optionally
d) silver,
for use in the treatment of psoriasis.

The effect of the compositions according to the present invention is greater than the sum of the effects obtained following separate administration of the individual ingredients of the combination. This superior effect is apparently due to synergy between the various ingredients.

The combination of the invention acts as a light compression bandage with an anti-exudative effect that accelerates wound healing.

The inclusion in the combination of the invention of a steroidal surfactant known to have a lipid-dissolving activity may seem paradoxical. However, in-depth studies have demonstrated that said ingredient, as well as performing an anti-infection activity, also aids the penetration of numerous medicaments.

It has been observed that topical administration of the composition in question to damaged tissues increases the subcutaneous concentrations of the surfactant components due to a synergic activity.

The composition of the invention will contain the active ingredients within the following weight ranges:
a) carnitine: 0.1 to 10.0 g,
b) sodium cholate: 0.1 to 10.0 g,
c) sodium acetate: 0.1 to 10.0 g,
   and possibly
d) silver: 0.01 to 0.1 g.

The composition of the invention will preferably contain the active ingredients within the following weight ranges:
a) carnitine: 4.0 to 6.0 g,
b) sodium cholate: 4.0 to 6.0 g,
c) sodium acetate: 4.0 to 6.0 g,
   and possibly
d) silver: 0.05 to 0.08 g.

The composition of the present invention can be formulated suitably for oral administration, and will be prepared by conventional methods well known in pharmaceutical technology, such as those described in Remington's Pharmaceutical Handbook, Mack Publishing Co., N.Y., USA.

Examples of formulations of the invention are set out below.

### Example 1 - Gel formulation

**GEL FORMULA**

| **INCI description** | **%** | **Function** |
|---|---|---|
| AQUA | <100 | Solvent |
| PVP | 12.00 | Film-forming/viscosity regulator |
| CARNITINE | 4.00 | Energy promoter/moisturising agent |
| SODIUM CHOLATE HYDRATE | 2.00 | Surfactant |
| GLYCERIN | 2.00 | Moisturising agent |
| SODIUM ACETATE TRIHYDRATE | 2.00 | Disinfectant/preservative |
| XANTHAN GUM | 1.00 | Film-forming/viscosity regulator |
| PHENOXYETHANOL | 0.50 | Preservative |
| BENZYL ALCOHOL | 0.30 | Disinfectant/preservative |
| TOCOPHERYL ACETATE | 0.10 | Antioxidant |
| BENZALKONIUM CHLORIDE | 0.10 | Disinfectant/preservative |
| CI 77820 | 0.05 | Disinfectant/preservative |

### Example 2 - Spray formulation

**SPRAY FORMULA**

| **INCI description** | **%** | **Function** |
|---|---|---|
| AQUA | <100 | Solvent |
| CARNITINE | 4.00 | Energy promoter/moisturising agent |
| POLOXAMER 407-PPG12/SMDI COPOLYMER | 3.00 | Film-forming/viscosity regulator |
| SODIUM ACETATE TRIHYDRATE | 2.00 | Disinfectant/preservative |
| SODIUM CHOLATE HYDRATE | 2.00 | Surfactant |
| PHENOXYETHANOL | 0.60 | Preservative |
| BENZYL ALCOHOL | 0.30 | Disinfectant/preservative |
| BENZALKONIUM CHLORIDE | 0.10 | Disinfectant/preservative |

## Claims

1. Composition comprising:
a) carnitine,
b) sodium cholate,
c) sodium acetate,
for use in the treatment of psoriasis.

2. Composition according to claim 1, comprising silver as a further ingredient.

3. Composition according to claim 1, comprising the active ingredients within the following weight ranges:
a) carnitine: 0.1 to 10.0 g,
b) sodium cholate: 0.1 to 10.0 g,
c) sodium acetate: 0.1 to 10.0 g.

4. Composition according to claim 3, comprising the active ingredients within the following weight ranges:
a) carnitine: 4.0 to 6.0 g,
b) sodium cholate: 4.0 to 6.0 g,
c) sodium acetate: 4.0 to 6.0 g.

5. Composition according to claim 3 comprising from 0.01 to 0.1 g of silver.

## Patentansprüche

1. Zusammensetzung, umfassend:
a) Carnitin,
b) Natriumcholat,
c) Natriumacetat
zur Verwendung in der Behandlung von Psoriasis.

2. Zusammensetzung nach Anspruch 1, die als weiteren Bestandteil Silber umfasst.

3. Zusammensetzung nach Anspruch 1, die die aktiven Bestandteile innnerhalb der folgenden Gewichtsbereiche umfasst:
a) Carnitin: 0,1 bis 10,0 g,
b) Natriumcholat: 0,1 bis 10,0 g,
c) Natriumacetat: 0,1 bis 10,0 g.

4. Zusammensetzung nach Anspruch 3, die die aktiven Bestandteile innerhalb der folgenden Gewichtsbereiche umfasst:
a) Carnitin: 4,0 bis 6,0 g,
b) Natriumcholat: 4,0 bis 6,0 g,
c) Natriumacetat: 4,0 bis 6,0 g.

5. Zusammensetzung nach Anspruch 3, die 0,01 bis 0,1 g Silber umfasst.

## Revendications

1. Composition comprenant :
a) de la carnitine,
b) du cholate de sodium,
c) de l'acétate de sodium,
pour une utilisation dans le traitement du psoriasis.

2. Composition selon la revendication 1, comprenant de l'argent comme autre ingrédient.

3. Composition selon la revendication 1, comprenant les ingrédients actifs dans les plages de poids suivantes :
a) carnitine : 0,1 à 10,0 g,
b) cholate de sodium : 0,1 à 10,0 g,
c) acétate de sodium : 0,1 à 10,0 g.

4. Composition selon la revendication 3, comprenant les ingrédients actifs dans les plages de poids suivantes :
a) carnitine : 4,0 à 6,0 g,
b) cholate de sodium : 4,0 à 6,0 g,
c) acétate de sodium : 4,0 à 6,0 g.

5. Composition selon la revendication 3 comprenant 0,01 à 0,1 g d'argent.
